(19) Europäisches Patentamt
European Patent Office
Office européen des brevets

(11) **EP 3 437 177 B1**

(12) **EUROPEAN PATENT SPECIFICATION**

(45) Date of publication and mention
of the grant of the patent:
**20.03.2024 Bulletin 2024/12**

(21) Application number: **17712626.5**

(22) Date of filing: **08.03.2017**

(51) International Patent Classification (IPC):
*H02K 49/10* (2006.01)     *H02K 1/14* (2006.01)
*H02K 5/128* (2006.01)     *B01F 33/45* (2022.01)

(52) Cooperative Patent Classification (CPC):
**H02K 49/108; B01F 33/45; H02K 1/146;
H02K 5/128**

(86) International application number:
**PCT/US2017/021412**

(87) International publication number:
**WO 2017/172316 (05.10.2017 Gazette 2017/40)**

(54) **MAGNETIC MIXER DRIVE FOR A BIOREACTOR**

MAGNETMISCHER FÜR EINEN BIOREAKTOR

MÉLANGEUR MAGNÉTIQUE POUR BIORÉACTEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.03.2016 US 201615087712
31.03.2016 US 201615087656
27.05.2016 US 201615166397**

(43) Date of publication of application:
**06.02.2019 Bulletin 2019/06**

(60) Divisional application:
**24156493.9**

(73) Proprietor: **Global Life Sciences Solutions USA
LLC
Marlborough, MA 01752 (US)**

(72) Inventors:
• **ALEXANDER, James Pellegrino
Niskayuna
New York 12309 (US)**
• **GEBAUER, Klaus
751 84 Uppsala (SE)**
• **TORREY, David Allan
Niskayuna
New York 12309 (US)**
• **ATALLA, Ashraf Said
Niskayuna
New York 12309 (US)**
• **DIDARI, Sima
Niskayuna
New York 12309 (US)**
• **DAMREN, Richard Lee
Marlborough
Massachusetts 01752 (US)**

(74) Representative: **Bedford, Grant Richard
Global Life Sciences Solutions Operations UK
Ltd
Amersham Place
Little Chalfont, Buckinghamshire HP7 9NA (GB)**

(56) References cited:
**EP-A1- 1 188 474      EP-A1- 2 813 281
CN-A- 105 327 639      JP-A- 2008 086 115
US-A1- 2012 228 978      US-A1- 2015 259 638**

**Description**

FIELD

Embodiments relate generally to the field of bioreactors, and more particularly to a magnetic mixer drive for a bioreactor.

BACKGROUND

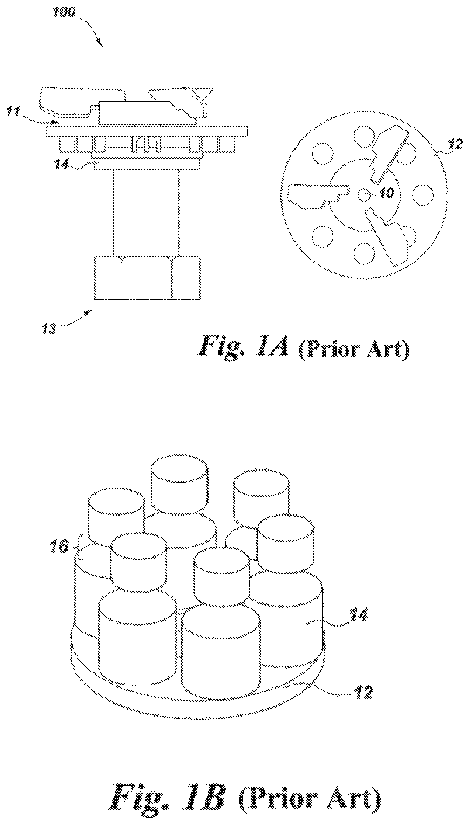

Mixers and pumps have a wide range of applications including bioreactors. The main elements in a rotary mixer 100 (FIG. 1A) are the drive 14 (which contains the driving mechanism) and the impeller 12 (which contains the mixing blades), at the impeller end 11. The two elements are aligned and coupled together by different topologies. In one aspect, the magnetic coupling is where the impeller 12 holds a set of magnets 10 that are coupled to the drive end 13. This is established by a set of magnets in the drive end rotated by a separate motor. The existing technology contains a set of cylindrical magnets 14 (*See* FIG. 1B) on each end separated by a certain "magnetic" gap 16 which is primarily composed of air, fixing elements, and mixing bag wall which are all non-magnetic elements. The existing technology suffers many impediments, including, but not limited to: (1) the weak coupling between the magnets which requires using more expensive and larger magnets (e.g., Neodymium magnets), (2) poor volume utilization, (3) the use of rare-earth magnets in the impeller which is a single use element that increases the cost of the impeller and poses environmental challenges, and (4) large drive size, as it constructs of a set of large coupling magnets driven by a separate electrical motor.

Mixing systems often include an agitator or impeller mechanically connected to a drive shaft lowered into a fluid through an opening in the top of a vessel. The drive shaft is connected to an electric motor arranged outside the vessel. In a closed vessel, a fluid seal is provided between the drive shaft and the wall of the vessel to prevent leakage of fluid from the vessel. Other mixing systems include a rotating magnetic drive head outside of the vessel and a rotating magnetic impeller as an agitation element within the vessel. The movement of the magnetic drive head enables torque transfer and thus rotation of the magnetic impeller allowing the impeller to mix and agitate the fluid within the vessel. Because there is no need in a closed vessel to have a drive shaft penetrate the vessel wall to mechanically rotate the impeller, magnetically coupled systems can eliminate the need for having fluid seals between the drive shaft and the vessel. Magnetic coupling of an impeller inside the vessel to a drive system or motor external to the vessel can eliminate contamination issues, allow for a completely enclosed system, and prevent leakage.

Increasingly, in the biopharmaceutical industry, single use or disposable containers or vessels are used as close type systems, typically in range of about 1 - 2000 liters. The vessel may be a tank-type support with for example substantially cylindrical shape and is made of rigid material such as stainless steel to provide sufficient support for the flexible bag or container, for example of a kind used in single-use bioreactors. Use of sterilized disposable bags eliminates time consuming steps of cleaning of the vessel and reduces the chance of contamination. The flexible container or bag is placed inside the vessel in an accurate manner so that for example different pipelines or tubes, mixers and sensors can be connected to the bag properly and accurately.

Combining the single use or disposable bags with a magnetic agitator system establishes a sterile environment that is utilized in biopharmaceutical manufacturing. A variety of vessels, devices, components and unit operations for mixing and manipulating liquids and/or for carrying out biochemical and/or biological processes are available. For example, biological materials including mammalian, plant or insect cells and microbial cultures can be processed using bioreactors that include single-use processing bags. Manufacturing of complex biological products such as proteins, monoclonal antibodies, etc. requires, in many instances, multiple processing steps ranging from fermentation or cell culture (bacteria, yeast, insect, fungi, etc.), to primary recovery and purification.

It is desirable to address the needs as stated above by utilizing less expensive elements that are more environmentally friendly. Aspects of the invention will run a much smaller impeller, and have a reduced magnetic force that will allow the bag to be separated from the drive more easily. Further, moving parts on the drive (user) end will be addressed to provide safer mechanisms.

The movement of the magnetic drive head enables torque transfer and thus rotation of the magnetic impeller allowing the impeller inside the vessel to mix and agitate the fluid within the vessel without providing a sealed shaft. The magnetic mixing principle is especially advantageous when using completely closed vessels, or when utilizing containers as required to maintain sterility of the internal volume and the fluid to be mixed.

In single-use processing technology, as employed in the production of biopharmaceuticals, plastic containers and bags are used which are typically pre-sterilized (e.g., by gamma irradiation), and employed as completely closed systems connected to adjacent fluid processing equipment and lines using aseptic connections. In these applications of single-use mixing vessels and bioreactors, the use of magnetic mixing technology is preferred for reasons of process safety, simplicity and the lower cost that comes by omitting complex sealing arrangements around rotating shafts.

Today, certain challenges are imposed on processes employing magnetic mixing technology where a direct and permanent mechanical connection between impeller and external drive by a shaft is lacking. These deficiencies include not knowing the actual speed of the impeller; and the torque and power input are more difficult to assess compared with a direct mechanical coupling. Further, as the power transferred by magnetic couplings is generally limited compared to mechanical shafts, magnetic mixers are typically operating at lower power input which makes it difficult to assess power input and torque on the background of frictional forces, disturbances and noise in such measurements. Therefore, there is a need to improve the assessment, measurement and control of magnetic mixing and magnetic mixer couplings.

In more details, challenges with current magnetic mixers include: (1) indirect (not real-time) determination of power delivered to the fluid, as performed with user-interface manipulation of formulas or look-up tables; (2) fluid density and/or viscosity changes as the mixing process takes place, without accurate control of the mixing process; and (3) inability to identify abnormalities in the mixing process. No feature or direct process in bioreactors used to date can detect or flag such issues. Various magnetically driven systems and devices are known. See, for example, EP 1 188 474 A1, US 2015/259638 A1, JP 2008 086115 A, CN 105 327 639 A and EP 2 813 281 A1. Moreover, no existing solution provides for a direct measure of the power delivered to the fluid while mixing. Prior methods have been dependent on look-up tables to calculate the power delivered to fluid. In addition, no device or method has been able to continuously monitor the fluid viscosity and density or to detect abnormalities in mixing process without the look-up tables as suggested prior.

It is desirable to address the needs as stated above by providing additional functionality to a bioreactor and/or mixer. It will allow accurate monitoring of the power delivered to the fluid while mixing, and will provide more accurate control by a user. It will also beneficially permit continuous updates of the fluid properties, and preferentially, alarms in cases of abnormalities in mixing, such as, for example, in the circumstance of 'flooding' of impellers when the ratio of volumetric gas to liquid ratio exceeds a threshold.

## SUMMARY

The invention is a bioreactor comprising a magnetic mixer drive as defined in the independent claim 1. Further embodiments of the invention are defined in the dependent claims 2 - 8.

The claimed invention pertains to a bioreactor mixer that strengthens the magnetic coupling to provide higher torque and replace the drive-end magnets and drive motor. Embodiments disclosed herein use a back iron on both ends to strengthen the magnetic coupling as well as pie-shaped magnets ("pie" shaped in the sense of a wedge shape and format (i.e., triangular/trapezoidal have a wider outer edge and smaller inner dimension, or shaped as a circular or annular sector) to increase the volume utilization and hence provide higher torque and allow the use of less expensive material (e.g. ferrites). In another embodiment, the rotor side is constructed with a Halbach array which increases the torque without the need to add a back iron piece. An axial flux stator is implemented to replace the drive-end magnets and the drive motor.

Embodiments of the invention may comprise a torque sensor, to detect the different fluid and mixing properties, conditions, and abnormalities in a mixing process. The torque produced in the mixing process relates to different fluid properties such as viscosity and density. It also relates to different mixing conditions such as presence of obstacles and changes or issue with gas sparging. Moreover, torque measurements enable determination of power transmitted to a fluid by actual measurement, in contrast to using solely empirical impeller power number and speed, and allow actual mass transfer determination (i.e., gas transfer calculations).

Embodiments thus provide additional functionality to a user of the bioreactor. Accurate monitoring of power delivered to the fluid while mixing is now possible, in real-time, allows continuous updates and adjustments as to the fluid properties. Alarms are implemented, as desired, in cases of abnormalities in the mixing process.

Detailed descriptions of various embodiments and further examples, not part of the claimed invention, are described as follows.

## BRIEF DESCRIPTION OF THE DRAWINGS

FIG. 1A (PRIOR ART) illustrates the magnetic drive for a bioreactor mixer.

FIG. 1B (PRIOR ART) illustrates a magnetic coupling where the impeller holds a set of magnets that are coupled to the drive end, the set of magnets in the drive end rotated by a separate motor.

FIG. 2 (PRIOR ART) depicts a cross-sectional view of the magnetic field coupling in a prior art system.

FIG. 3 depicts a cross-sectional view of the magnetic field coupling when using the individual magnet shapes and configuration as disclosed in embodiments herein.

FIG. 4A illustrates an example, not part of the claimed invention, including a back iron on both ends.

FIG. 4B illustrates an example, not part of the claimed invention, including pie-shaped magnets, individually positioned, angled, and spaced in a magnetic arrangement around a central axis.

FIG. 4C illustrates the rotor side constructed with a Halbach array in another example, not part of the claimed invention, the individual magnets adjacent one another without spacing to form a concentric magnetic arrangement.

FIG. 5 depicts a cross-sectional view showing the magnetic coupling in embodiments disclosed herein when using the pie-shaped magnets and back iron.

FIG. 6 provides a comparison of magnets as utilized in embodiments of the invention.

FIG. 7 depicts a comparison of maximum torque for cylindrical magnets using different materials in one embodiment.

FIG. 8 depicts a comparison of maximum torque for cylindrical magnets with back iron.

FIG. 9 depicts a comparison of maximum torque for pie-shaped magnets and Halbach array

FIG. 10 depicts a graphic illustration of the relationship of torque versus current density for the axial flux stator in one embodiment.

FIG. 11 illustrates an embodiment of the invention from a perspective side view.

FIG. 12 illustrates an example, not part of the claimed invention, from a perspective side view.

FIG. 13 (a), (b), (c) shows analyses of varying the stator length.

FIG. 14 (a), (b), (c) shows an analysis of varying the current density.

FIG. 15 (a), (b), (c) demonstrates analyses of varying the slot pitch, and resulting torque.

FIG. 16A illustrates a perspective view of an embodiment of a tooth wound axial flux stator.

FIG. 16B depicts a perspective top view of the axial flux stator of FIG. 16A.

FIG. 16C depicts a perspective side view of the axial flux stator of FIG. 16A.

FIG. 16D depicts a perspective side view of the tooth height of the axial flux stator of FIG. 16A.

FIG. 16 E depicts an embodiment of a stator core described herein.

FIG. 17 is an illustration that demonstrates as the Reynolds Number decreases, a point is reached where the power number begins to increase sharply, as dependent on the type of impeller utilized.

FIG. 18 charts power number, $N_p$, versus Reynolds Number, $N_{re}$.

FIG. 19 depicts the variation of impeller torque as a function of rotating speed for Fluids 1, 2, 3, and water.

FIG. 20 illustrates the variation in torque-speed slope (dT/dn) for different viscosities, as shown by Fluids 1, 2, 3, and water.

FIG. 21A shows the variation of impeller torque as a function of rotating speed for Fluids 4, 5, 6, and water, whereby the viscosity is kept constant and density is varied to 1.1X, 1.3X, and 1.5X, respectively.

FIG. 21B shows the variation in torque-speed slope for different fluid densities in one example.

FIG. 22A is a perspective view a printed circuit board (PCB) winding incorporated between an arrangement of magnets to pick up the back electro-motive force (EMF).

FIG. 22B is a graphical depiction of the torque versus load angle.

FIG. 22C is an example a flux sensor, here a printed circuit board (PCB) winding, that is utilized to acquire flux information and estimate torque.

FIG. 23 is a perspective view of an embodiment of an axial flux stator with the magnetic arrangement, and assembled with an impeller portion.

FIG. 24 is a bioreactor drive and impeller comprising 12 magnets on each, between which the magnetic flux or the magnetic field density sensor(s) are placed.

FIG. 25A shows the variation in the axial and the transverse magnetic field densities when measured on the top of the drive magnet, using a magnetic sensor, at different impeller positions.

FIG. 25B shows the variation in the axial and the transverse magnetic field densities when measured 0.125" (3.2 mm) from the top of the drive magnet, using a magnetic sensor, at different impeller positions.

FIG. 25C shows the variation in the magnetic flux when measured by a loop placed on the top circumference of the magnet, using PCB winding or pick-up coil, at different impeller positions.

FIG. 26 illustrates the mixing process in a schematic defining the implementation of one or more sensor(s) with the mixing system.

FIG. 27 illustrates a flexible bioreactor bag with a rotating magnetic impeller, mounted in a rigid support vessel with a magnetic drive.

## DETAILED DESCRIPTION

Various embodiments will be better understood when read in conjunction with the appended drawings.

The embodiments disclosed pertain to a bioreactor mixer that strengthens the magnetic coupling to provide higher torque and replace the drive-end magnets and/or drive motor, as desired. Embodiments include pie-shaped magnets ("pie" shaped in the sense of a wedge shape and wedged format (i.e., triangular/trapezoidal have a wider outer edge and smaller inner dimension, or shaped as a circular or annular sector), such that the wedges fit together to increase the volume utilization and hence provide higher torque, and further allow the use of less expensive material (e.g. ferrites).

In one embodiment, the rotor side is constructed with a Halbach array which increases the torque. Embodiments disclosed may utilize a back iron on one or both ends to strengthen the magnetic coupling. With the Halbach array, torque is increased without a back iron piece. An axial flux stator is implemented to replace the drive-end magnets and the drive motor. Embodiments are disclosed as follows.

Disposable bioreactors

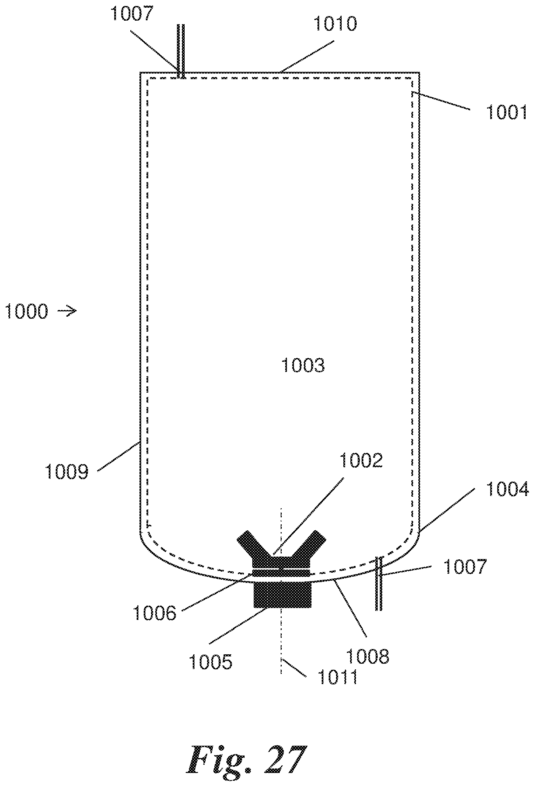

All the embodiments disclosed are applicable to a disposable bioreactor 1000 as illustrated in Fig. 27. The bioreactor comprises a flexible bag 1001 as the bioreactor vessel, with a magnetic impeller 1002 in an inner volume 1003 of the bag. The bag is suitably configured to be mounted in a rigid support vessel 1004, comprising a magnetic drive 1005 for the impeller, such that the impeller is driven by the magnetic drive and rotates around a central axis of rotation 1011 with a speed adequate for agitating the content of the bioreactor. The bag comprises a receiver structure 1006 for receiving the impeller and aligning it with the magnetic drive (suitably along axis 1011), where the receiver structure may e.g. comprise a fixed shaft or a cavity capable of receiving an end of a rotating impeller shaft or part of a rotating impeller. The inner volume of the bag can e.g. be 20-5 000 liters, such as 50 - 2 000 liters. The bag may have one or more ports 1007 for introduction and removal of materials such as e.g. cell culture media, cell inoculates, cell culture samples, nutrients, gases and/or exhaust gas. Suitably, the bag containing the impeller is supplied presterilized, e.g. by gamma radiation sterilization. Although the magnetic drive is here shown mounted in a bottom wall 1008 of the support vessel, it may also be mounted in a side wall 1009 or a top wall 1010 of the support vessel. The impeller and any receiver structure should then be placed at a corresponding position of a bag wall.

Impellers

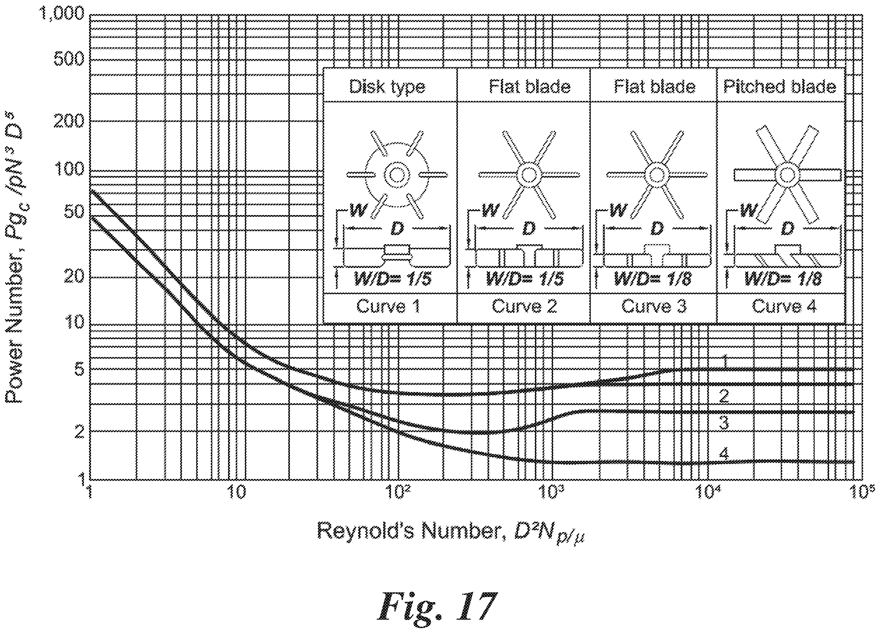

In all the mixing/mixer system, flexible bioreactor bag and embodiments discussed, the impeller is a rotating magnetic impeller driven by a magnetic drive. The impeller is suitably capable of rotating around a central axis of rotation, which is suitably aligned with an axis of the drive. The impeller may rotate around a fixed shaft comprised in the system, but it may equally well comprise a shaft fixed to the impeller (or integral with the impeller) such that the shaft co-rotates with the impeller. Such a shaft may rotate in a cavity or bearing comprised in the system, typically forming a part of an impeller receiver structure. The impeller may comprise one or more mixing blades, e.g. as illustrated in Fig. 17.

Pie-Shaped Magnet Configuration

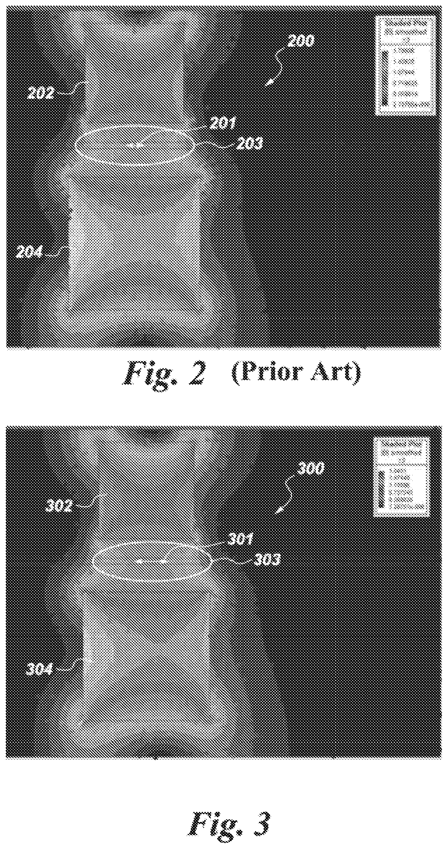

For comparison purposes, FIG. 2 depicts a cross-sectional view of the magnetic field coupling 201 in a prior art system between a drive end magnet 204 and an impeller end magnet 202; The magnetic field density 203 is the area represented there between. An embodiment disclosed herein is shown in FIG. 3 where pie-shaped magnets are utilized in the mixer 300. A cross-sectional view shows a large increase in the magnetic field coupling 301, the increased magnetic field density 303 between the drive end magnet 304 and impeller end magnet 302.

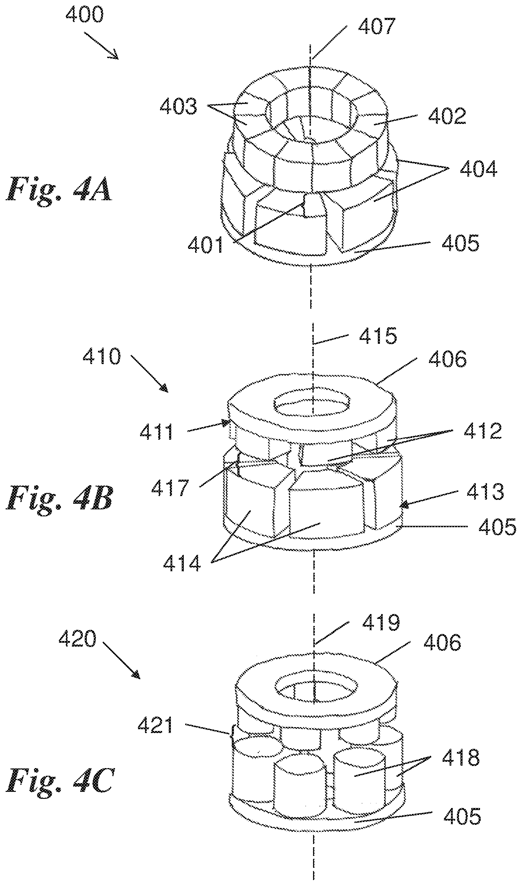

FIG. 4A depicts the rotor side of a mixer 400 with a Halbach array 402 in one example, not part of the claimed invention. The individual magnets 403 are adjacent one another without spacing to form a concentric magnetic arrangement of the Halbach array 402 around a central (rotational) axis 407. The pie-shaped drive end magnets 404 are spaced apart and positioned on a magnetic plate (back plate) 405. The magnetic plate may be a back iron, any magnetic material, including but not limited to any material of magnetic permeability greater than air. In one aspect, the magnetic plate may be a high magnetic permeability alloy such as an alloy comprising cobalt, iron, or other magnetic material. A magnetic field density 401 is created by the space remaining where the individual magnets 403 of the Halbach array 402 levitate above the drive end magnets 404.

Back Iron at Impeller and Drive Ends

FIG. 4B illustrates an example, not part of the claimed invention, of a magnetic drive 410 including wedged (pie-shaped) magnets 412 at a drive end 413 and wedged (pie-shaped) magnets 414 at an impeller end 411, each magnet 412, 414 individually positioned, angled, and spaced in a magnetic arrangement around a central (rotational) axis 415. A magnetic plate 405 is positioned at the impeller end 411 while a second magnetic plate 406 is positioned at the drive end 413. The magnetic plates 405, 406 are a back iron, in one embodiment, and may be any magnetic material, including but not limited to any material of magnetic permeability greater than air. A magnetic field density 417 is created between the drive end magnets 414 and impeller end magnets 412, thus increasing the magnetic coupling. In another aspect, the magnetic plate is a high magnetic permeability including materials including, but not limited to, cobalt, iron, or other magnetic material.

In one comparative example, not part of the claimed invention, , as shown in FIG. 4C, a magnetic drive 420 includes an impeller end back iron 406 and drive end back iron 405. The cylindrical shaped magnets 418 are spaced around a central (rotational) axis 419, and positioned at each end such that a magnetic field density 421 is created therebetween. The back irons enhance the magnetic coupling of the magnetic drive, thereby increasing torque.

As illustrated in FIG. 4C, back irons are placed in the drive above the impeller end magnets and below the drive end magnets. The back irons reduce the overall magnetic path in air, the magnetic field density 421, between the drive end and the impeller end magnets. As a result, the magnetic field density that couples the two ends increases.

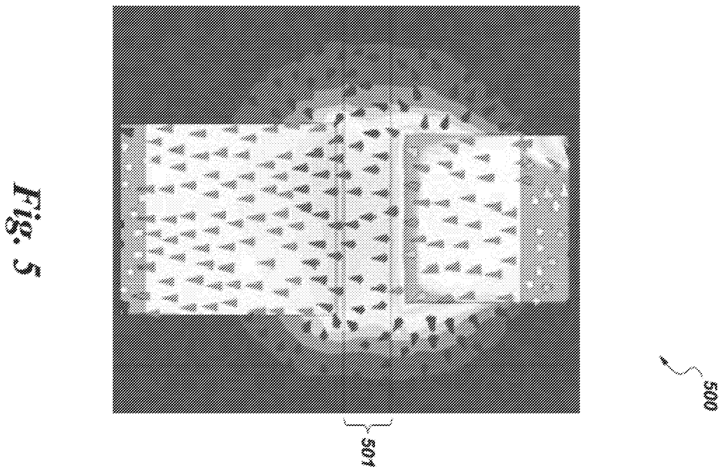

FIG. 5 depicts a cross-sectional view of a mixing system 500 showing the magnetic coupling 501 in embodiments disclosed herein when using the pie-shaped magnets and back irons, as described in *FIG. 4B.*

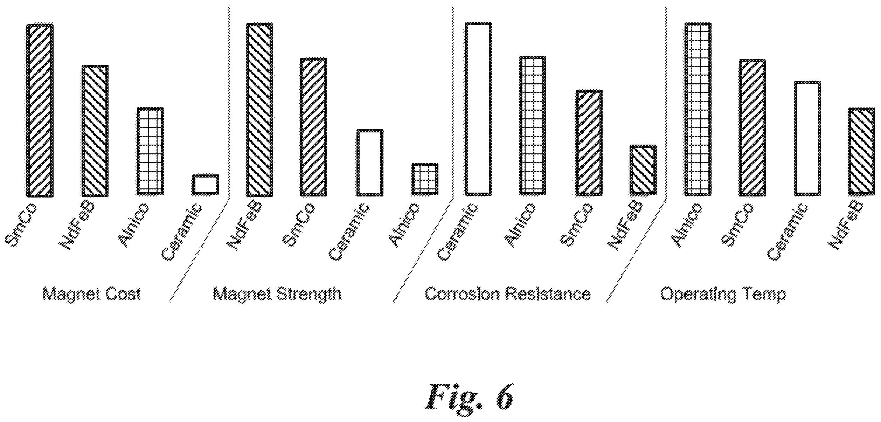

A comparison of magnets is shown in FIG. 6. Baseline torque is at about 5.8 Nm with cylindrical shaped magnets as shown in *FIG. 4C.*

Table 1: Materials used for the magnets in the magnetic drive.

| [0086] Material | | [0087] Grade | [0088] Magnet price (USD/kg) |
|---|---|---|---|
| [0089] Rare earth | [0090] Sintered NdFeB | [0091] N42SH | [0092] 70 |
| | [0093] SmCo | [0094] 28/7 | [0095] 87 |
| [0096] Non-rare earth | [0097] Alnico | [0098] Alnico 9 | [0099] 57 |
| | [00100] Ferrite | [00101] CC8B/AC12 | [00102] 8.5 |

FIG. 6 illustrates that non-rare earth magnet materials, e.g., Alnico 9 and C8B/AC12 (ceramic) are cheaper and have beneficial characteristics that align with the desired magnetic compositions, characteristics, and configurations described. Non-rare earth magnets may here and throughout the following be defined as magnets comprising less than 5 wt % of rare earth elements, such as less than 1 wt% of rare earth elements. Rare earth elements comprise elements of the lanthanide series (i.e. the elements with atomic numbers 57-71) as well as scandium and yttrium.

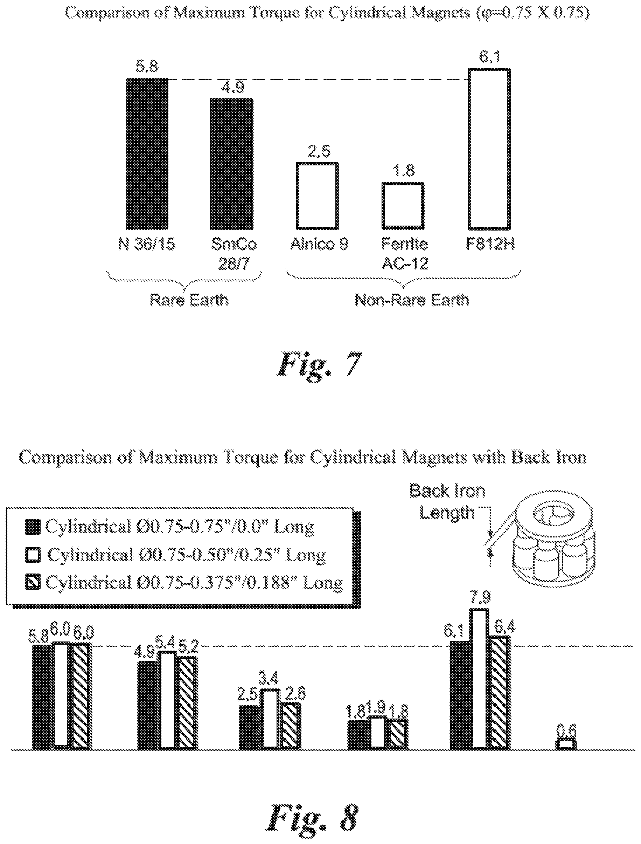

FIG. 7 is a comparison of maximum torque for cylindrical magnets ($\phi$ = 0.75 x 0.75). By replacing Neodymium (Sintered NdFeB) magnets, and using other non-rare earth magnets, while keeping the cylindrical shapes and arrangement, the torque production is greatly reduced.

In one aspect, a back iron may be added on one of the impeller end or the drive end. The back iron added at impeller and drive ends improves torque production, as illustrated in FIG. 8. Specifically, FIG. 8 demonstrates the comparison of maximum torque for cylindrical magnets with back iron, as represented in *FIG. 4C.* FIG. 8 shows that maximum torque produced by different types of magnets (e.g., N 38/15, SmCo 28/7, etc.) as a function of magnet geometry. The ferrite magnet grade FB12H gives twice the amount of the torque produced without the back plate, while eliminating the use of rare earth magnets. Three of the magnet grades are not able to produce the baseline torque of the rare earth magnets.

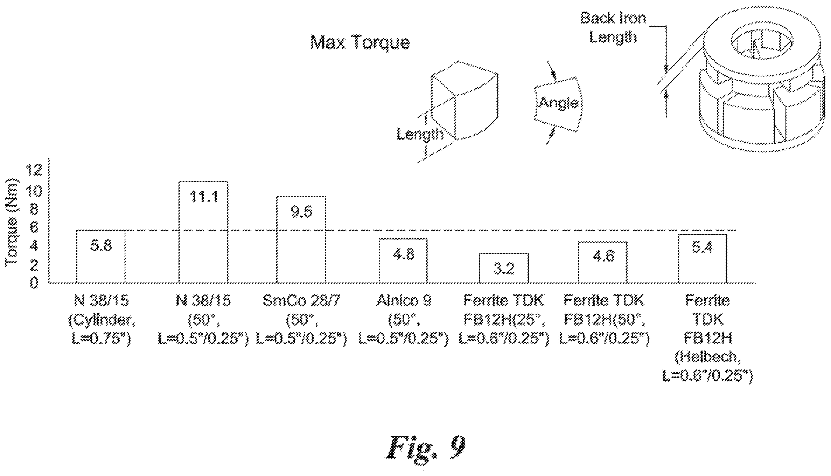

Embodiments of the invention modify the shape of the magnets from cylindrical to pie-shaped configurations. As shown in FIG. 9, the improved impeller-drive design provides 2.5 times the torque without the need for expensive Neodymium magnets. Ferrite magnet grade FB12H gives 2.5 times the amount of torque produced without the back plate and the magnet pie shape. As demonstrated in *FIG. 4B,* and referenced in FIG. 9, a comparison is shown of maximum torque for pie-shaped magnets.

Halbach Magnet Array

As shown in FIG. 9, an embodiment of the Halbach impeller-drive design of *FIG. 4A* provides the desired torque without the need for expensive Neodymium magnets. This arrangement of Halbach array and pie shaped magnets increases the torque produced by a factor of 3 which delivers the baseline torque.

Axial Flux Stator

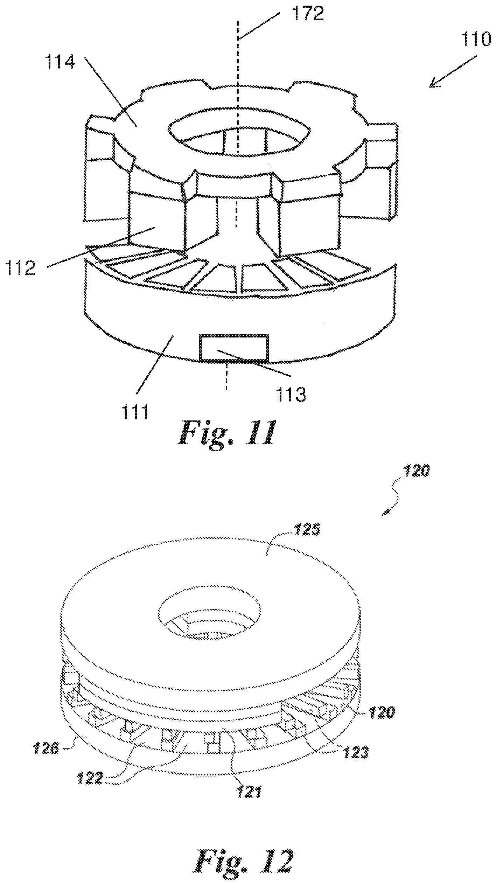

The invention provides an axial flux stator to reduce the drive end size, as well as reducing the number of components, and increase its reliability. FIG. 11 depicts an embodiment of the axial flux stator 110 without the shaft and bearing, and without the lever mechanism as utilized in the prior art. The drive stator 111 is positioned on an underside of the magnets 112 of the impeller end 114, aligned along a central axis of rotation 172, and has a control circuit 113.

The electrical and mechanical components of the axial flux stator 110 are adjusted to address challenges in the modified design. In embodiments of the axial flux stator, the electrical components comprise: high current density (cooling), a higher number of slots to allow high count of slots per pole and hence the ability to choose various coil span (to suppress harmonics), longer slots resulting in higher leakage inductance and lower power factor, and higher current results in higher flux thus accommodating a bigger stator tooth to avoid saturation.

The construction of an axial flux stator 120 is depicted in FIG. 12 as an axial-type magnetic gear. (Example, not part of the claimed invention). A magnetic core 121 is here shown positioned between a high-speed rotor 125 (stator with high frequency) and a low speed rotor 126. The magnetic core 121 has a number of slots 122 depending on the arrangement of the impeller magnets and windings. The magnetic core may be laminated, powder type, or a taped core, as desired. A set of windings are shown in FIG. 12 as stationary steel segments 123, or stator teeth, with modulation slots 122. The set of windings may be tooth-wound, distributed, or fractional windings, as well. Windings can also be single or multi-layer, wave or lap windings, full or short pitched windings. The stationary steel segments can have additional slotting to accommodate magnetic gearing (e.g., Vernier type machine) with single or multi-stage gearing. The stator can also have additional magnets when using a Vernier type machine.

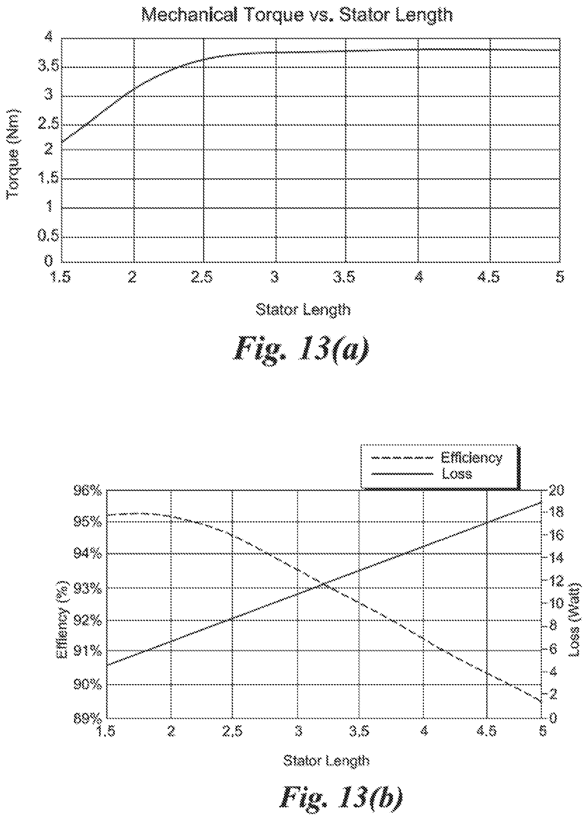

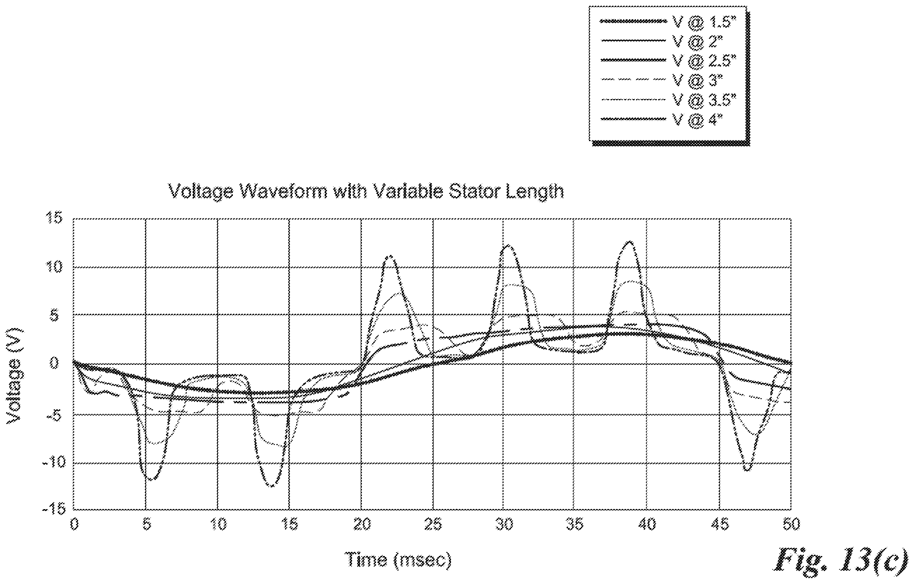

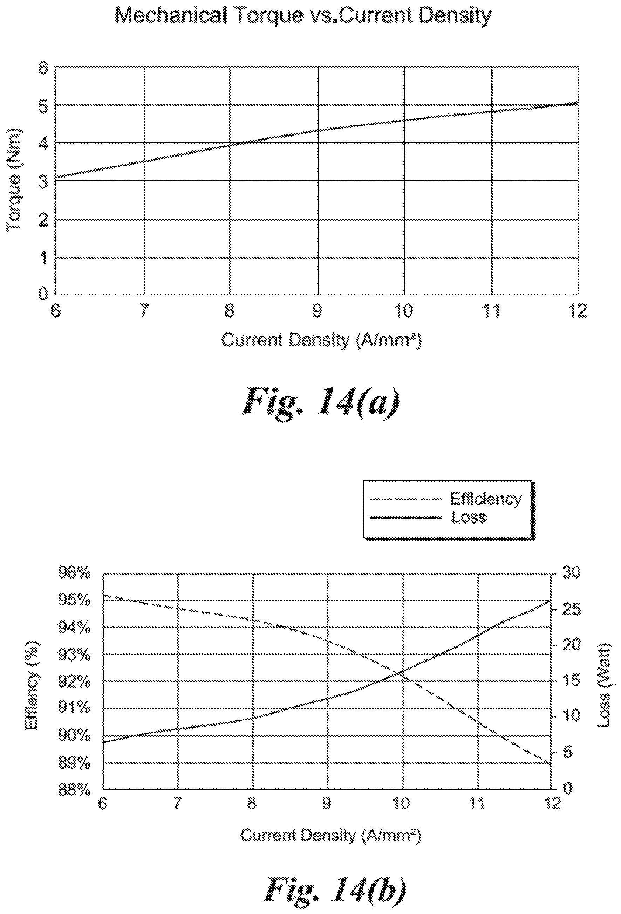

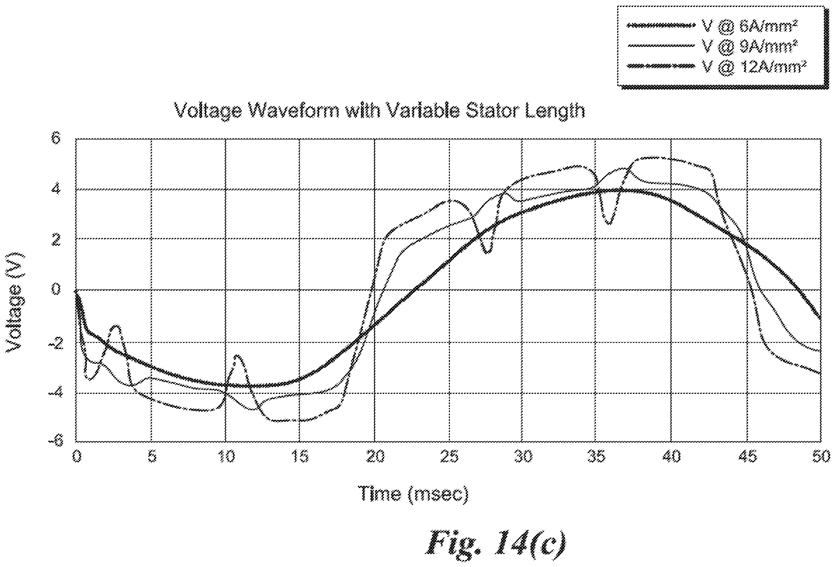

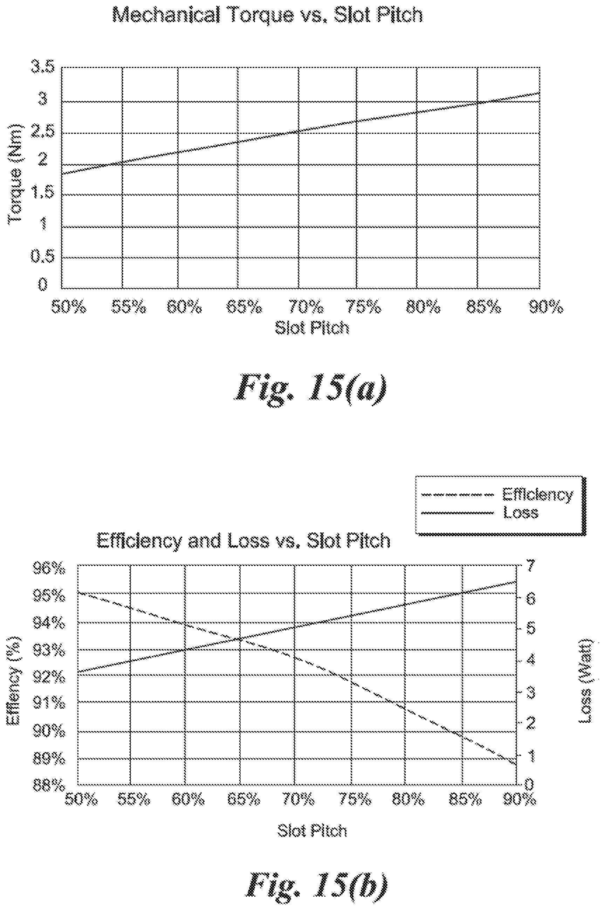

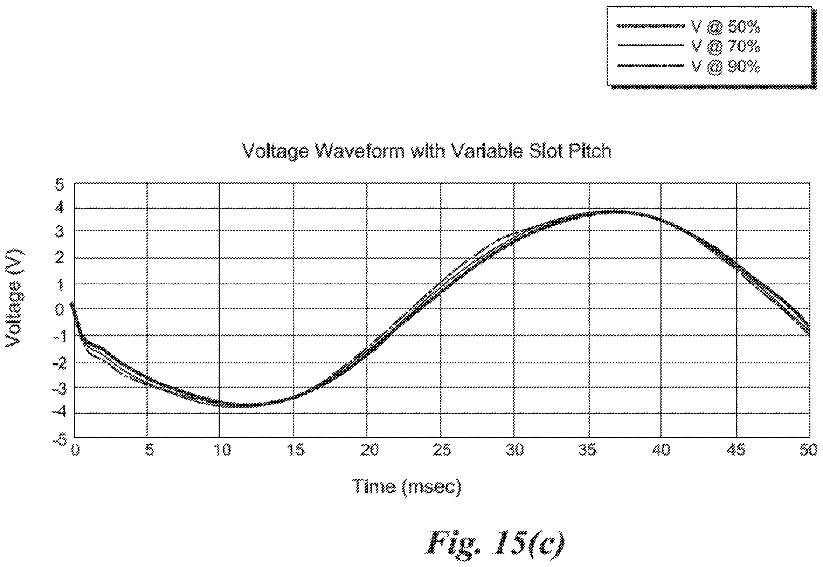

An analysis of the axial flux stator with distributed windings (e.g., an eighteen-slot example) demonstrates that by increasing the stator length, a higher current path results per slot; saturation of the teeth occurs and causes increased harmonics (*See FIG. 13*). Increasing the stator current density results in higher torque; again, saturation of the teeth occurs and causes increased harmonics (*See FIG. 14*). And, by increasing the stator slot pitch, higher torque results while placing mechanical constraints on the innermost tooth width (*See FIG. 15*).

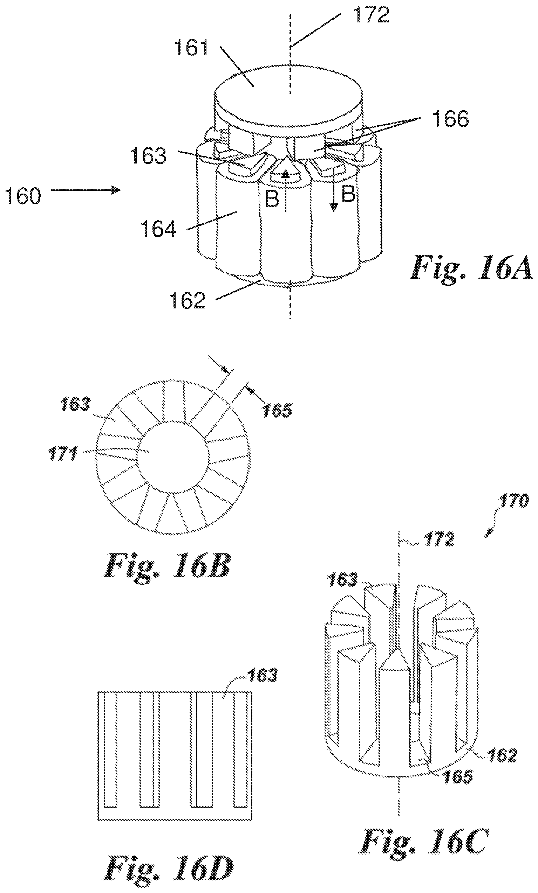

FIG. 16A illustrates an embodiment of the claimed invention, of a tooth wound axial flux stator 160 with nine (9) slots 165 (*See FIG. 16C*), for exemplary purposes, and not limitation. FIGS. 16B, 16C, and 16D depict perspective views to more clearly illustrate the details of the axial flux stator 160. The stator core comprises pie-shaped magnetic stator teeth 163 that extend vertically from the stator back iron 162. The stator core 170 can be formed from sintered powdered iron, ferrite, or machined from a coil of magnetic steel. Conductive windings (current carrying elements) 164 are wound around the stator teeth 163. The conductive windings are divided up into phases. Within each phase winding, the sense, field direction, of the individual coils alternates so that the application of phase current to the phase winding creates a magnetic field that is directed vertically upward in one tooth and vertically downward in another tooth. The flow of current through the conductive windings forms a magnetic field that flows through the stator teeth, across the air gap between the stator 160 and a rotor 161 capable of rotating around central axis 172, interacts with the magnets 166 on the rotor, travels through the rotor 161, and returns through a rotor magnet of opposite magnetic polarity, across the air gap between the stator and rotor, through an oppositely-excited stator tooth, closing through the stator back iron 162.

As shown in FIG. 16B, the exemplary stator has nine slots 165 of width of about 0.470in (1.2 cm); the inside diameter (a hollow core 171 with central axis 172) of the stator core 170 is about 1.575in (4.0 cm), and the outside diameter of the stator core is about 3.250in (8.25 cm). The width of the slots, the core inside diameter, and core outside diameter are parameters that determine the performance of the motor. FIG. 16C depicts the magnetic stator core 170 including stator teeth 163 in combination with the base back iron 162 and shows that the stator core is about 2.750in (7.0 cm) height, but can vary in size, shape and dimension, as desired for a particular mixer. FIG. 16D depicts the height of the magnetic pie-shaped stator teeth 163, the height of which is shown here as about 2.500in (6.35 cm). The height of the stator teeth and the thickness of the stator back iron are additional parameters determined during the design of the stator, and so may be varied in shape and dimension.

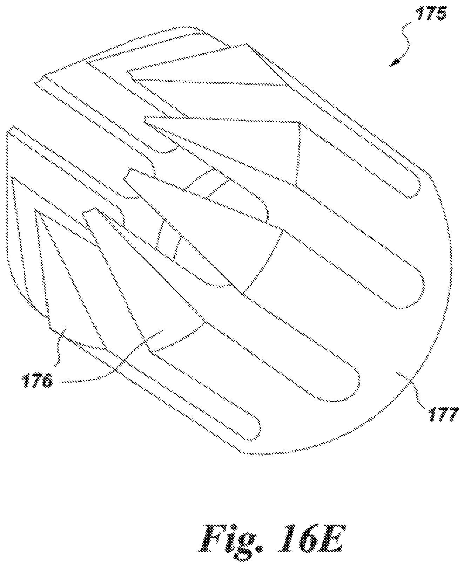

In one embodiment, as shown in FIG. 16E, the stator core 175 is defined with conic teeth 176 and including a back iron 177. As such, the stator core may be modified and designed to provide operational efficiency of a mixer.

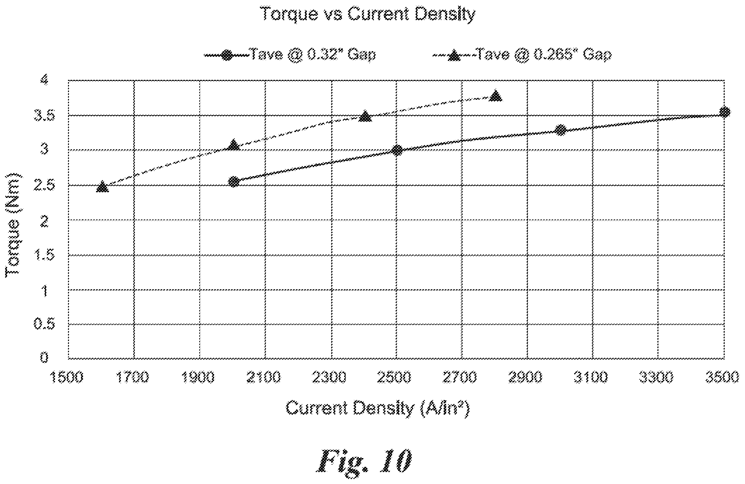

FIG. 10 compares torque versus current density of the axial flux stator 160 for gaps of about 0.32 inches (0.81 cm) and about 0.265 inches (0.67 cm) between the stator and rotor. More torque is produced for a given current density with the smaller gap. The efficiency is slightly greater for the smaller gap since the current is smaller.

As demonstrated, the embodiments and examples thus described address the problems presented in the art. The cheaper impeller as described comprises a magnetic coupling improved by increasing the magnetic field density. Higher torque density is produced by optimizing the impeller magnet by way of material, shape, and back iron, individually or in combination. The rare earth magnets can be replaced by less expensive and environmentally friendly non-rare earth materials, such as Alnico and Ferrite, for example. In addition, the large and oversized drive assembly used prior now can run a much smaller impeller. Further, the moving parts on the drive side, near the user have been repositioned to provide a safer device overall.

Additional technical and commercial advantages are provided with the more reliable system that includes the axial flux stator; the axial flux stator has a smaller drive, no moving parts, and reduced magnetic force during bag installation. While cost and availability of the magnets is an advantage with improving magnet shape and material, the efficiency of the magnetic coupling that increase torque is very useful for further intensification of the bioreactor, various mixing systems, and overall efficiency.

In use, for example, microbial fermentations utilize more agitation for sufficient mixing, gas mass transfer and heat transfer at the reactor wall. An improved device of the invention including the axial flux stator improves user experience due to a less complex design. Not only is the design more compact than a drive with permanent magnets, but the installation and removal of the bag is improved as there are no permanent magnetic forces that have to be overcome. Moving parts are avoided with consequences on machinery directive requirements, sealing and ingress protection to avoid prior standard design that utilized a lever mechanism to separate the drive from the impeller such that the bag can be pulled out of the reactor bottom end piece.

Torque produced in the mixing process relates to different fluid properties such as viscosity and density. Torque also relates to different mixing conditions such as presence of obstacles and changes or issue with gas sparging. For example, disruption of the continuous liquid or sparger liquid by zones of air presents large bubbles or channels, a behavior typically called [gas] flooding of the mixer; this leads to a drastic reduction of power input. A torque measurement (i.e. a continuous measurement in real time) under conditions close to or at the point of flooding of the mixer allows for better process control and higher utilization of mixing power, including improved capacity and capability in the process step. For example, a bioreactor could operate at higher mass transfer and thus higher productivity. Moreover, torque measurements, along with speed, enable determination of power transmitted to fluid by actual measurement, in contrast to using solely empirical impeller power number and speed, and thus allow actual mass transfer determination (i.e., gas transfer calculations). As described herein, examples refer to a torque and speed sensor, such as a transducer, and a method of using the measured torque and speed to detect the different fluid and mixing properties, conditions, and abnormalities.

Mixing Power

Examples, not part of the claimed invention, are disclosed which allow the power consumed by a rotating impeller to be easily measured in a process fluid. The units express this power as 'horsepower' (HP). Mixer performance relates to horsepower; problems, however are associated with this tendency. In general, Power (P) input to the fluid can be calculated for typical mixers (turbulent flow) applications as follows:

$$P = \frac{\rho N_p N^3 D^5}{g_c} \quad \text{(Eq 1)}$$

$\rho$ = Specific Gravity
$N_p$ = Power Number of Impeller
$N$ = Impeller Speed
$D$ = Impeller Diameter
$g_c$ = dimensional constant

Viscosity Effect

As viscosity increases, the impeller power number may begin to increase. This becomes important in the HP calculations because as power number begins to go up so does the horsepower utilized to drive the mixer. Simply increasing the input [horse-] power may be the answer, but this change reduces the service factor of the mixer drive, hence a 'bigger' mixer may be required. Viscosity increase also affects the flow characteristics of fluid as compared to water.

Reynold's Number

**[0048]** Reynolds Number is a dimensionless number that can be derived as follows:

$$N_{re} = \frac{D^2 N \rho}{\mu} \quad \text{Eq (2)}$$

$\rho$ = Fluid Density
$\mu$ = Viscosity

The power number ($N_p$) is constant for each impeller type, as long as the Reynolds number is sufficiently high. The power number is a function of Reynolds Number ($N_{re}$).

The illustration of FIG. 17 shows how the power number for each impeller varies with changes in Reynolds Number. As the Reynolds Number drops, a point is reached where the power number begins to increase sharply. This point depends on the type of impeller in use. Reynolds Numbers or $N_{re}$ between 1000 and 2000 are generally considered "in transition".

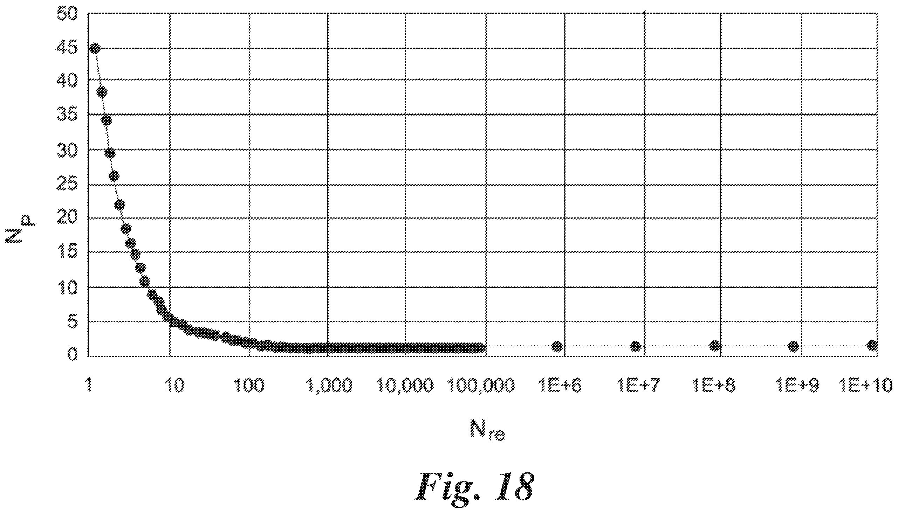

The Reynolds number is the indicator of the type of mixing fluid in which the mixer will operate. If the Reynolds Number is above 2,000, the power number is constant, When the Reynolds Number calculated is less than 1,000 (i.e., laminar flow), then the power number increases as the Reynolds Number decreases. Consequently, the shaft horsepower calculated is based on the corrected power number. In this case, as shown in FIG. 18, a power number ($N_p$) vs Reynolds Number ($N_{re}$) curve is obtained from the impeller manufacturer or by experimentation.

The power utilized to mix a fluid at a given speed can vary based on multiple parameters, including but not limited to: (i) the impeller diameter, (ii) the impeller blade design, (iii) the fluid properties (i.e. viscosity and density). In some applications, such as mammalian cells mixers, controlling the power delivered to the fluid is an element of the mixing process. Since the mixing power is driven by the drive system, it can be measured and controlled from that side. The drive system can be in the form of a stator that rotates the impeller, or a set of magnets coupled to the impeller magnets and driven by a separate motor. In these embodiments, the magnetic field that couples the drive to the impeller depends on the power (and also torque) delivered to the impeller. By measuring the magnetic field or flux, the torque, speed, and power delivered to the impeller can be calculated and hence controlled. Further, the current and voltage inputs to the drive system are related to the power delivered to the system. These values can also be used to calculate the power delivered to the impeller.

Characterizing a Fluid

Fluid properties of density $\rho$ and viscosity $\mu$ play a role in specifying the desired mixing power and torque, as these properties are represented in the Reynolds number calculation as well as in the specific power equation. In estimating such parameters, for exemplary purposes, and not limitation, curve #4 from the FIG. 17 is shown in FIG. 18.

Next, the torque-speed curves are plotted for seven different fluids: water, and six other fluids with different density, dynamic viscosity, or combination of both, than water. Table 1 shows the general properties of each fluid (at 25°C) while Table 2 shows the impeller and tank properties.

Table 1

| Property | Water | Fluid #1 | Fluid #2 | Fluid #3 | Fluid #4 | Fluid #5 | Fluid #6 |
|---|---|---|---|---|---|---|---|
| Fluid Density, $\rho$ [kg/m$^3$] | 1000 | 1000 | 1000 | 1000 | 1100 | 1300 | 1500 |
| Dynamic Viscosity, $\mu$ [Pa.s] | 0.00089 | 0.0089 | 0.089 | 0.89 | 0.00089 | 0.00089 | 0.00089 |

Table 2

| Property | Value |
|---|---|
| Impeller Diameter [in] | 3.5 |
| Tank Volume [Lit] | 200 |

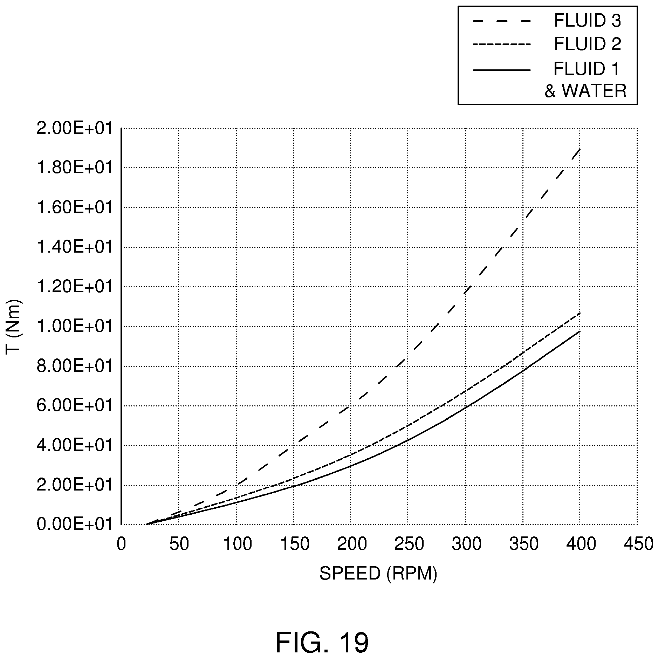

The calculation for the torque-speed characteristics are done using equation 1 (Eq. 1) and equation 2 (Eq. 2). FIG. 19 shows the variation of impeller torque as a function of rotating speed for Fluids 1, 2, 3, and water. The viscosity is the variable parameter in this calculation. Fluid 1 does not show detectable variation from water, while Fluid 2 and Fluid 3 show differences due to the increase in viscosity (100 times for Fluid 2 and 1000 times for Fluid 3).

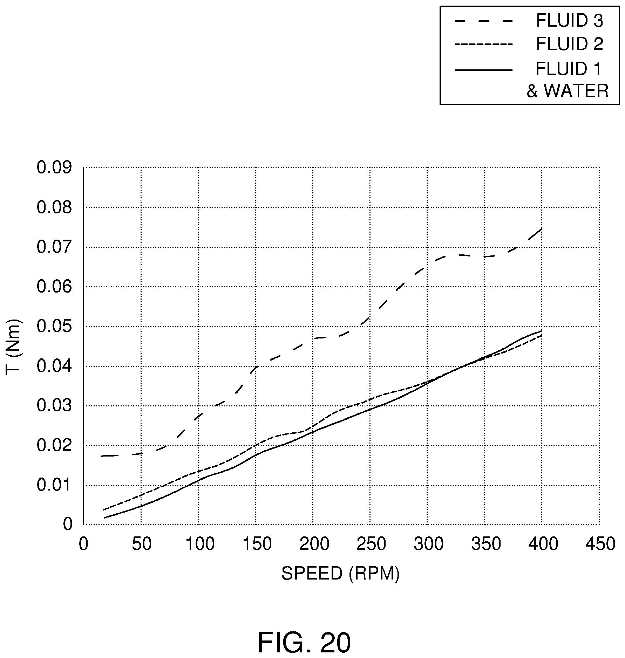

FIG. 20 shows the variation in torque-speed slope for different viscosities. The variation in slope is minimal when varying the viscosity, while the variation in y-axis crossing point is differentiated. Again, Fluids 1, 2, 3, and water are plotted; Fluid 1 does not show detectable variation from water.

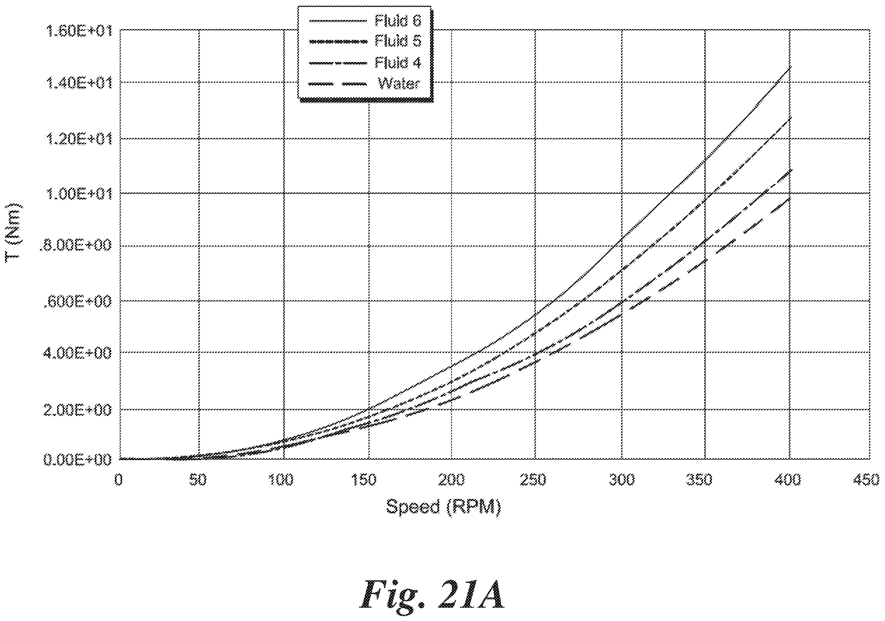

FIG. 21A shows the variation of impeller torque as a function of rotating speed for Fluids 4, 5, and 6 as compared to water. The viscosity is kept constant and density is varied (relative to the density of water) to 1.1X, 1.3X, and 1.5X, respectively. The variation of density is detectable for the fluids.

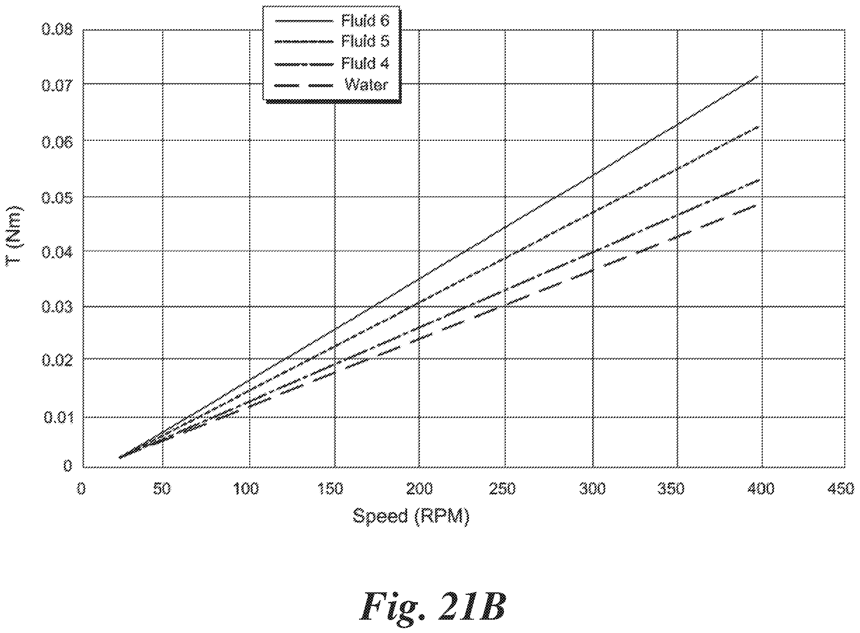

FIG. 21B shows the variation in torque-speed slope for different fluid densities of Fluids 4, 5, and 6 as compared to water. While the variation in slope due to viscosity is minimal, the density effects the slope, and minimally, the y-axis intercept.

Hence, following the relationships outlined above, the change of viscosity in a fluid may be detected by measuring the impeller torque at different speeds. In addition, changes in density are detectable at various levels. By studying the torque-speed slope, the variation in fluid properties can be distinguished between variations in viscosity or density.

Examples below (not part of the claimed invention) describe the method of measuring the torque for bioreactors and various types of mixers.

To measure the torque and speed of an impeller, transducers can be installed on the shaft, in the space between the impeller and drive, on the impeller magnets, or on the drive magnet or core as additional components.

Method 1: Measuring Torque and Speed as Related to Magnet-Magnet Coupling

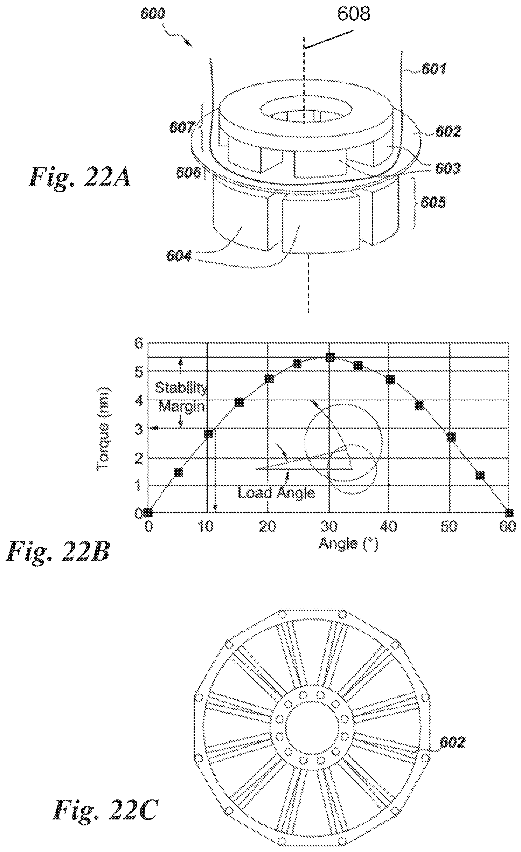

Examples, not part of the claimed invention, include a sensor positioned outside a bag or vessel, outside the closed system, that does not allow for electrical wiring inside the bag. In one aspect, the sensor is integrated with the drive head. FIG. 22A depicts a system 600 with a printed circuit board (PCB) winding 602 incorporated with an arrangement of magnets 603, 604 of an axial flux stator 605. The system 600 includes a first set of magnets 603 at the impeller end 607 (rotatable around central axis 608), the impeller end positioned within a vessel 601, and a second set of magnets 604 positioned at the drive end 605. The PCB winding 602 is a single coil, or set of coils as shown in greater detail of FIG. 22C, and placed between the sets of magnets 603, 604 in the area 606 where the magnetic gradient is arranged. FIG. 22B demonstrates that synchronous torque depends on load angle, such that the angle between the rotor and the stator fluxes (i.e., the angle between the rotor pole (or magnet) and the stator pole (or magnet)). By placing a single coil or a set of coils, such as the printed circuit board (PCB) winding 602, between the stator 605 and the rotor 607, the magnetic flux in the space or area 606 between the drive and the impeller (partially or fully filled with air) can be detected and related to the produced torque. In one aspect, a single coil or the set of coils are printed on a circuit board, and can be arranged and placed in a single layer or multi-layers.

A flux sensor, such as the PCB winding 602 (shown in FIG. 22C) is installed on an existing magnet-magnet coupling of a bioreactor, or mixer system, to acquire flux information and estimate torque. The flux sensor functions to acquire the speed by relating the measured voltage to the speed of rotation. The voltage, as it changes with time, is measured at various instances. In FIG. 22C, the illustration depicts a PCB winding 602 that picks up the back electromotive force (EMF).

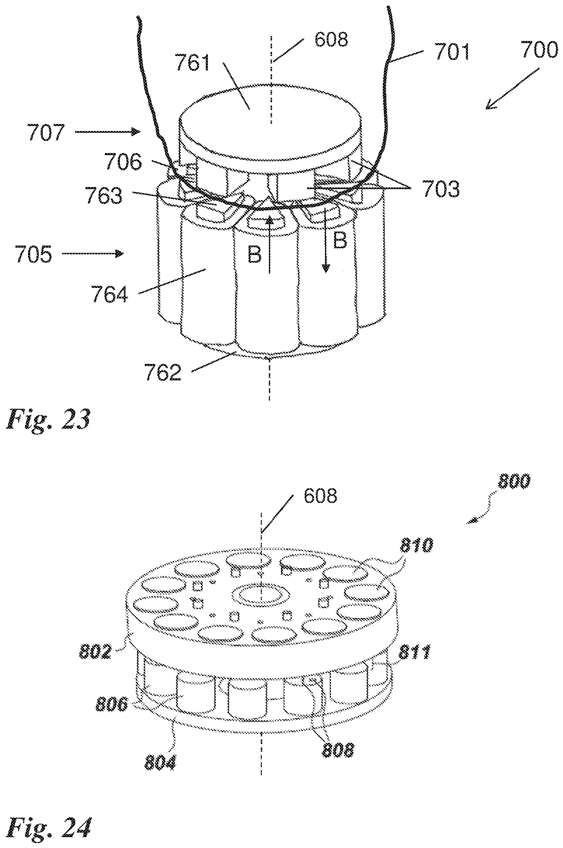

A magnetic field density sensor 808 (e.g., one or more 3D hall-effect based sensors, anisotropic magnetoresistance (AMR), shingled magnetic recording (SMR), giant magnetoresistance (GMR) sensors), and shown in FIG. 24, is installed in the space between the drive and the impeller. The magnetic field density in this space changes as the torque produced by the impeller changes. FIG. 24 depicts a magnet-magnet coupling bioreactor system 800, using integrated sensors 808 to measure the varying viscosity and varying density when the system is in use. Impeller magnets 810 at the impeller end 802 form a first portion and the drive-end magnets 806 incorporate with a base steel plate 804 form a second portion. As depicted, sensors 808, including magnetic field density sensors, are integrated with a drive magnet 806. The sensors, however, may be incorporated, integrated, and/or placed within any region of the system 800. Specifically, the sensors shown are integrated within the region 811 between the impeller-end magnets and the drive-end magnets. The produced torque relates to the fluid properties (e.g., weight, volume, viscosity, density). At speed $n \rightarrow dT/dn$, $T$ is used to identify fluid viscosity, density, and different operating conditions.

Method 2: Measuring Torque and Speed as Related to Axial Flux (AF) Stator

With the axial flux stator, as shown in FIG. 23, the stator voltages and currents are acquired and decomposed to direct and quadrature axis components, and back EMF and torque are calculated without the need for sensors. FIG. 23 illustrates an embodiment of a device 700 having a first rotor portion 707 positioned within a vessel 701, and a second stator portion 705; the second stator portion is a tooth wound axial flux stator 705 that comprises pie-shaped magnetic stator teeth 763 that extend vertically from the stator back iron 762. The stator core can be formed from sintered powdered iron, ferrite, or machined from a coil of magnetic steel. Conductive windings (current carrying elements) 764 are wound around the stator teeth 763. The conductive windings are divided into phases. Within each phase winding, the field direction of the individual coils alternates so that the application of phase current to the phase winding creates a magnetic field (B) that is directed vertically upward in one tooth and vertically downward in another tooth. The flow of current through the conductive windings forms a magnetic field that flows through the stator teeth, across the air gap, or region 706 between the stator 705 and a rotor 761, interacts with the magnets 703 on the rotor, travels through the rotor 761, and returns through a rotor magnet 703 of opposite magnetic polarity, across the air gap between the stator and rotor, through an oppositely-excited stator tooth, closing through the stator back iron 762.

While Method 1 is described in terms of the magnet-magnet coupling system 800 and can be applied to several different arrangements of drive and impeller, Method 2 is specific for wound stator drive system 700 as shown in FIG. 23. The magnet-magnet coupling system leverages the change in magnetic field density and/or magnetic flux to produce information on the position of the impeller and hence, the produced torque and/or speed. The stator drive system acquires the input current and voltage to the wound stator and relates such information to the produced torque and/or speed.

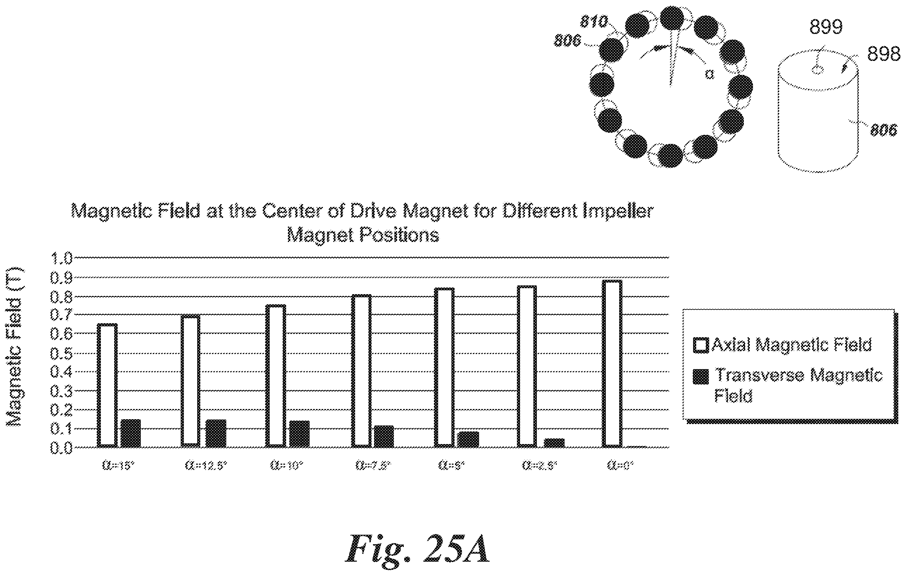

FIG. 25A shows a comparison of the magnetic field density acquired on the center point 899 of a top surface 898 of one drive magnet 806 (see FIG. 24) for different impeller positions. Impeller positions are recorded as the angle difference between the center line of the impeller magnets 810 and the corresponding center line of the drive magnet 806. The density of the magnetic field is recorded in both axial and transverse directions and it shows significant differences as the impeller-drive angle changes. These values are directly related to the produced torque and are used to deduce the produced torque.

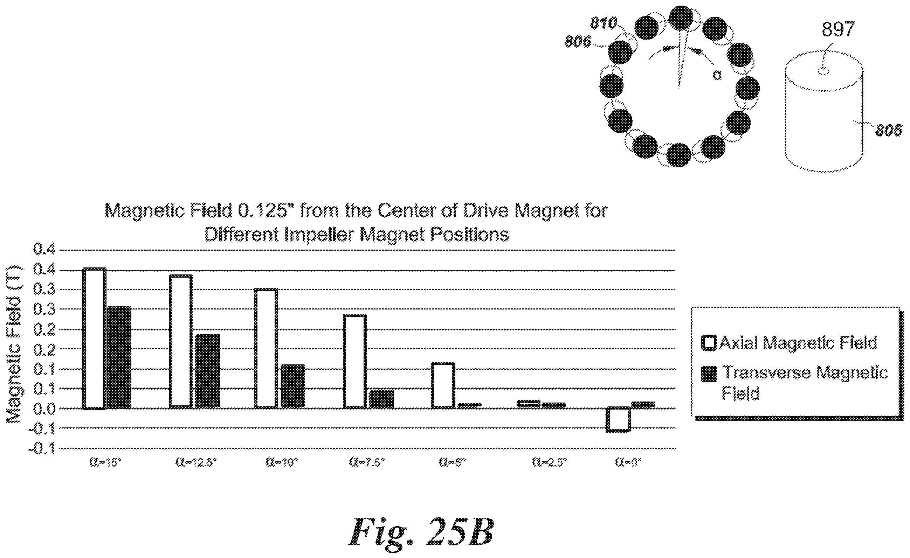

FIG. 25B is similar to figure FIG. 25A with the difference of the point of measurement. Here, the point of measurement 897 is shifted 1/8th of an inch (3.2 mm) in the axial direction towards the impeller, the point of calculation is 0.125" (3.2 mm) above the center 899 of the drive magnet 806. Again, the changes in magnetic field density is relative to changes in the impeller magnet 810 position. The sensor position can be anywhere between the drive and the impeller, or even on the bottom surface of the drive or the top surface of the impeller.

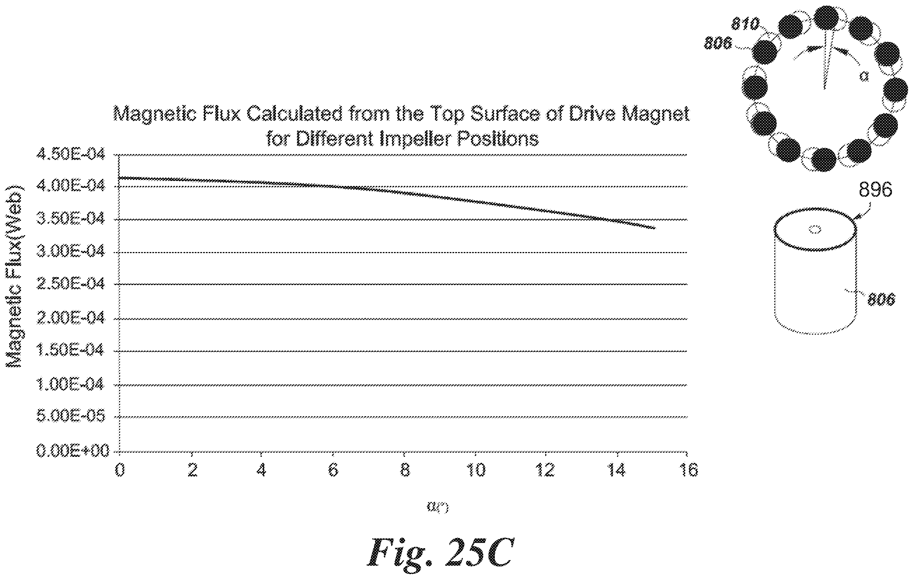

FIG. 25C shows the change in magnetic flux for different impeller positions. It is clear that the magnetic flux can also be used to detect the impeller position and hence the produced torque. Since the flux changes with impeller relative position, it can be used also to detect sudden impeller position, relative to drive, changes. This can be explained using Faraday's law:

$$e = -N \frac{d\phi}{dt}$$

Here, e is the produced voltage in the loop, used to pick-up the magnetic flux, N is the number of turns of the loop, and $\phi$ is the magnetic flux through the loop 896. A change in the impeller relative position causes a sudden change in the loop voltage (since the voltage is related to the time-change on the magnetic flux) and hence, this change in voltage can be related to the change in the impeller relative position. If the impeller speed increases over a certain time (t), then the voltage during this period can be used to calculate the new impeller relative position and speed. If the impeller changes relative position suddenly, due to an abnormal condition, then the voltage waveform is very short in time (more like a pulse) and hence an abnormality behavior can be detected and a subsequent action triggered.

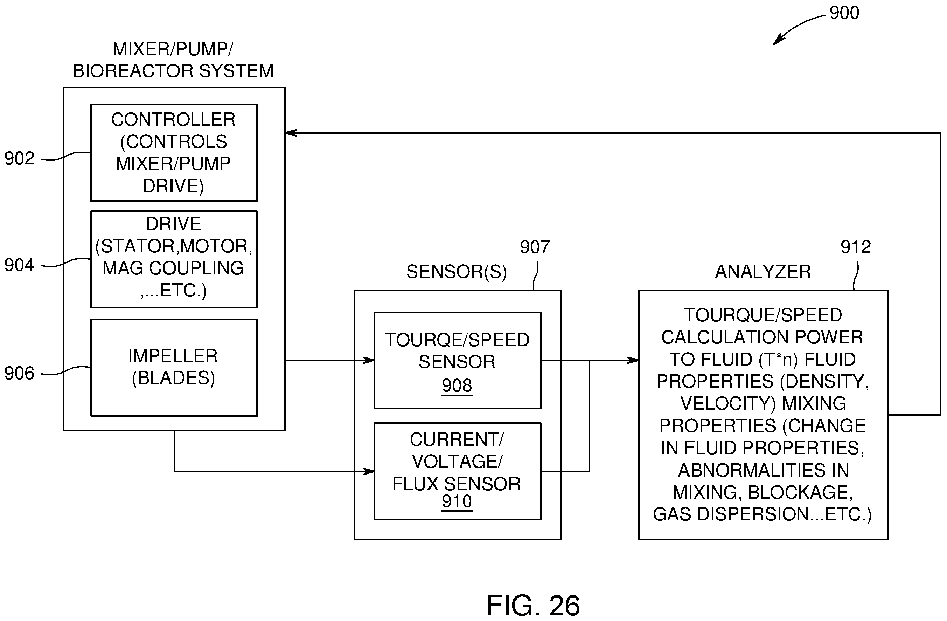

FIG. 26 is a schematic of a mixer system 900. The mixer system is a bioreactor system. The mixer 900 includes a controller 902; a drive 904 includes a stator, a motor, magnetic coupling, among other components; and an impeller 906 includes one or more blades, among other components. The controller 902 controls the mixer and/or pump drive 904. The drive may include any one of a stator, motor, magnetic coupling, alone or in combination. The sensor arrangement 907 includes a torque-speed sensor 908, a current/voltage/flux sensor 910, alone or in combination. The sensors 907 relay information to a processor 912 which analyzes the torque and speed calculation, analyzes power provided to the fluid, fluid properties (e.g., density, viscosity, etc.), mixing properties (e.g., change in fluid properties, abnormalities in mixing, blockage, gas dispersion, etc.), and other analyses as selected. The processor then provides direction to the controller 902 in a feedback loop. In this manner, the processor is an analyzer that provides precise control of the mixer to increase or decrease agitation, direct power into the system, adjust fluid properties, and correct any deficiencies, abnormalities, or otherwise.

Examples, not part of the claimed invention, include the assessment of the angle in between the drive and the impeller during mixing operations. This allows for determination of torque, viscosity, and other fluidic properties. This provides a common feature between the dedicated sensor (*See FIG. 22C, sensor 602*) and the indirect measurement with the axial flux stator.

The angle in between the drive and the impeller is determined by optical methods such that a marker on the impeller is read by an optical detection system. In such an embodiment, reflecting light from a fiber would allow ease of detection as the impeller is close to the bag bottom and a transparent window can fit with the bag. Other position indicators are possible as well.

In a further example, not part of the claimed invention, a discrete Hall sensor is utilized. The signal can be processed and compared against the position of the drive, in either a rotating drive or a flux stator. Calibration for a zero torque (offset) case without liquid or other conditions can also be configured. The use of a magnetic field sensor, direct or indirect, can thus be modified and altered in size, shape, and dimension as desired by a user.

Benefits include detection of fluid viscosity and density, as well as power and torque, delivered to the fluid inside the mixer. Obstructions are detected during start-up, including for example, sediment of micro-carriers, cells or undissolved powder in the bottom of the mixer. Torque measurements enable determination of power transmitted to fluid by actual measurement, in contrast to using solely empirical impeller power number and speed according to Eq. 1), hereby allowing for actual mass transfer determination (e.g., gas transfer calculations). In addition, flooding of the impeller in multiphase systems (e.g., gas sparged bioreactor) can be detected. Changes and any issues in gas sparging can be detected. Correct positioning of the disposable unit, and its impeller, can be verified. Measurement and monitoring of the different properties can also be used for the process analytical tool (PAT).

Further addressed are the challenges and issues that arise in the field. Determination of power delivered to the fluid is currently performed with formulas or look-up tables and not directly measured. Fluid density and/or viscosity changes as the mixing process takes place, thus, updated values provide accurate control of the mixing process. Abnormalities in mixing process, such as blockage, obstacles, or issues with gas sparging, may also be determined to ensure quality of the mixing process. These features detect and flag such issues, possibly even providing an alarm, so that the mixing process can be corrected.

Examples disclosed herein provide additional functionalities to the user of the bioreactor or mixer, as desired. Examples allow accurate monitoring of the power delivered to the fluid while mixing, and allow continuous updates on the fluid properties, including alarms in cases of abnormalities in mixing. Such examples may be modified so as to encompass features and components such as temperature, pressure, and other measurable conditions. The embodiments and examples disclosed herein may be incorporated with any size, shape, and dimension of vessel, bag, mixing container or otherwise.

It is to be understood that the above description is intended to be illustrative, and not restrictive. The scope of the invention is defined by the appended claims.

**Claims**

1. A bioreactor (1000) comprising a magnetic mixer drive, comprising:

   a flexible bag (1001);
   a magnetic impeller (1002) provided in an inner volume (1003) of the flexible bag (1001), said magnetic impeller (1002) being provided in a receiver structure (1006) for receiving the magnetic impeller (1002) and aligning it with the magnetic mixer drive along an axis (1011); and **characterized by**
   a magnetic drive (1005) comprising a stator (110;111;120;160;605;705) comprising a plurality of current carrying elements (164;764) for producing a magnetic flux in an axial direction along the axis (1011) for driving the magnetic impeller (1002) to rotate around said axis (1011); and
   wherein the stator is an axial flux stator having a core (121;170;175) to affix the current carrying elements (164;764), said core (121;170;175) including a plurality of stationary segments, each segment having height and a width with slots therebetween, the segments integrated on a plate with a hollow central axis, wherein the plurality of stationary segments are pie or conic shaped stator teeth (163;176;763) and the current carrying elements (164;764) are conductive windings wound around the stator teeth; and
   wherein the stator is mounted in a wall (1008; 1009; 1010) of a rigid support vessel (1004) and said flexible bag (1001) is adapted to be received in said rigid support vessel (1004).

2. The bioreactor (1000) of claim 1, wherein the magnetic impeller (1002) further comprises a plurality of magnets (404;414;418;603;810) and/or one or more mixing blades.

3. The bioreactor (1000) of claim 2, wherein said plurality of magnets (404;414;418;603;810) form a Halbach array, and/or wherein said magnetic impeller (1002) further comprises a back plate (405) to enhance the magnetic coupling.

4. The bioreactor (1000) of any preceding claim, wherein the plurality of stator teeth (163;176;763) are provided in a configuration around the axis (1011).

5. The bioreactor (1000) of any preceding claim, wherein at least one of the plurality of current currying elements (164;764) has a conic arrangement around the stator teeth (163;176;763).

6. The bioreactor (1000) of any preceding claim, wherein:

   a) the plurality of current carrying elements (164;764) in the stator are operable to control any one of impeller speed, impeller torque, and impeller power, alone or in combination; and/or
   b) a flow of current through the current carrying elements (164;764) in the stator is controllable to form a magnetic field that flows through the stator teeth (163; 176;763), across an air gap (706) between the stator and the magnetic impeller (1002) and back.

7. The bioreactor (1000) of any preceding claim, wherein the core (121;170;175) is magnetic.

8. The bioreactor (1000) of any preceding claim, further comprising a torque sensor therein.

**Patentansprüche**

1. Bioreaktor (1000), der einen magnetischen Mischerantrieb umfasst, umfassend:

   einen flexiblen Beutel (1001);
   ein magnetisches Laufrad (1002), das in einem Innenvolumen (1003) des flexiblen Beutels (1001) bereitgestellt ist, wobei das magnetische Laufrad (1002) in einer Aufnahmestruktur (1006) zum Aufnehmen des magnetischen Laufrads (1002) und zum Ausrichten desselben mit dem magnetischen Mischerantrieb entlang einer Achse (1011) bereitgestellt ist; und **gekennzeichnet durch**
   einen magnetischen Antrieb (1005), der einen Stator (110; 111; 120; 160; 605; 705) umfasst, der eine Vielzahl stromführender Elemente (164; 764) zum Erzeugen eines Magnetflusses in einer axialen Richtung entlang der Achse (1011) zum Antreiben des magnetischen Laufrads (1002) umfasst, um sich um die Achse (1011) zu drehen; und
   wobei der Stator ein Axialflussstator ist, der einen Kern (121; 170; 175) zum Befestigen der stromführenden Elemente (164; 764) aufweist, wobei der Kern (121; 170; 175) eine Vielzahl stationärer Segmente beinhaltet, wobei jedes Segment eine Höhe und eine Breite mit Schlitzen dazwischen aufweist, wobei die Segmente auf einer Platte mit einer hohlen Mittelachse integriert sind, wobei die Vielzahl stationärer Segmente tortenstück- oder kegelförmige Statorzähne (163; 176; 763) sind und die stromführenden Elemente (164;764) leitende Wicklungen sind, die um die Statorzähne gewickelt sind; und
   wobei der Stator in einer Wand (1008; 1009; 1010) eines starren Stützbehälters (1004) montiert ist und der flexible Beutel (1001) ausgeführt ist, um in dem starren Stützbehälter (1004) aufgenommen zu werden.

2. Bioreaktor (1000) nach Anspruch 1, wobei das magnetische Laufrad (1002) weiter eine Vielzahl von Magneten (404; 414; 418; 603; 810) und/oder einen oder mehrere Mischflügel umfasst.

3. Bioreaktor (1000) nach Anspruch 2, wobei die Vielzahl von Magneten (404; 414; 418; 603; 810) ein Halbach-Array bilden und/oder wobei das magnetische Laufrad (1002) weiter eine Rückplatte (405) zum Verbessern der magnetischen Kopplung umfasst.

4. Bioreaktor (1000) nach einem vorstehenden Anspruch, wobei die Vielzahl von Statorzähnen (163; 176; 763) in einer Konfiguration um die Achse (1011) herum bereitgestellt sind.

5. Bioreaktor (1000) nach einem vorstehenden Anspruch, wobei mindestens eines der Vielzahl stromführender Elemente (164; 764) eine konische Anordnung um die Statorzähne (163; 176; 763) herum aufweist.

6. Bioreaktor (1000) nach einem vorstehenden Anspruch, wobei:

   a) die Vielzahl stromführender Elemente (164; 764) in dem Stator betreibbar sind, um eines von der Laufradgeschwindigkeit, dem Laufraddrehmoment und Laufradleistung einzeln oder in Kombination zu steuern; und/oder
   b) ein Stromfluss durch die stromführenden Elemente (164; 764) in dem Stator steuerbar ist, um ein Magnetfeld zu bilden, das durch die Statorzähne (163; 176; 763) hindurch, durch einen Luftspalt (706) zwischen dem Stator und dem magnetischen Laufrad (1002) und zurück fließt.

7. Bioreaktor (1000) nach einem vorstehenden Anspruch, wobei der Kern (121; 170; 175) magnetisch ist.

8. Bioreaktor (1000) nach einem vorstehenden Anspruch, der weiter einen Drehmomentsensor darin umfasst.


**Revendications**

1. Bioréacteur (1000) comprenant un entraînement de mélangeur magnétique, comprenant :

   un sac flexible (1001) ;
   une turbine magnétique (1002) prévue dans un volume interne (1003) du sac flexible (1001), ladite turbine magnétique (1002) étant prévue dans une structure réceptrice (1006) destinée à recevoir la turbine magnétique (1002) et à l'aligner sur l'entraînement de mélangeur magnétique le long d'un axe (1011) ; et **caractérisé par**
   un entraînement magnétique (1005) comprenant un stator (110 ; 111; 120 ; 160 ; 605 ; 705) comprenant une pluralité d'éléments porteurs de courant (164 ; 764) destiné à produire un flux magnétique dans une direction axiale le long de l'axe (1011) pour entraîner la turbine magnétique (1002) à tourner autour dudit axe (1011) ; et

dans lequel le stator est un stator à flux axial présentant un noyau (121 ; 170 ; 175) pour fixer les éléments porteurs de courant (164 ; 764), ledit noyau (121 ; 170; 175) incluant une pluralité de segments fixes, chaque segment présentant une hauteur et une largeur avec des fentes entre eux, les segments étant intégrés sur une plaque avec un axe central creux, dans lequel la pluralité de segments fixes sont des dents de stator de forme circulaire ou conique (163 ; 176 ; 763) et les éléments porteurs de courant (164 ; 764) sont des enroulements conducteurs enroulés autour des dents de stator ; et

dans lequel le stator est monté dans une paroi (1008 ; 1009 ; 1010) d'un récipient de support rigide (1004) et ledit sac flexible (1001) est adapté pour être reçu dans ledit récipient de support rigide (1004).

2. Bioréacteur (1000) selon la revendication 1, dans lequel la turbine magnétique (1002) comprend en outre une pluralité d'aimants (404 ; 414; 418; 603 ; 810) et/ou une ou plusieurs pales de mélange.

3. Bioréacteur (1000) selon la revendication 2, dans lequel ladite pluralité d'aimants (404 ; 414 ; 418 ; 603 ; 810) forment un réseau de Halbach et/ou dans lequel ladite turbine magnétique (1002) comprend en outre une plaque arrière (405) pour améliorer le couplage magnétique.

4. Bioréacteur (1000) selon une quelconque revendication précédente, dans lequel la pluralité de dents de stator (163 ; 176 ; 763) sont prévues dans une configuration autour de l'axe (1011).

5. Bioréacteur (1000) selon une quelconque revendication précédente, dans lequel au moins un élément de la pluralité d'éléments porteurs de courant (164 ; 764) présente un agencement conique autour des dents de stator (163 ; 176 ; 763).

6. Bioréacteur (1000) selon une quelconque revendication précédente, dans lequel :

a) la pluralité d'éléments porteurs de courant (164 ; 764) dans le stator peuvent être actionnés pour commander l'un quelconque de la vitesse de turbine, du couple de turbine et de la puissance de turbine, individuellement ou en combinaison ; et/ou

b) un flux de courant à travers les éléments porteurs de courant (164 ; 764) dans le stator peut être commandé pour former un champ magnétique qui circule à travers les dents de stator (163 ; 176 ; 763), à travers un entrefer (706) entre le stator et la turbine magnétique (1002) et inversement.

7. Bioréacteur (1000) selon une quelconque revendication précédente, dans lequel le noyau (121 ; 170 ; 175) est magnétique.

8. Bioréacteur (1000) selon une quelconque revendication précédente, comprenant en outre un capteur de couple à l'intérieur de celui-ci.

*Fig. 1A* (Prior Art)

*Fig. 1B* (Prior Art)

*Fig. 2* (Prior Art)

*Fig. 3*

400

407

403

402

404

*Fig. 4A*

401

405

415

410

406

411

412

417

*Fig. 4B*

413

405

414

419

406

420

421

*Fig. 4C*

418

405

500

501

*Fig. 5*

Fig. 6

Comparison of Maximum Torque for Cylindrical Magnets (φ=0.75 X 0.75)

*Fig. 7*

Comparison of Maximum Torque for Cylindrical Magnets with Back Iron

*Fig. 8*

*Fig. 9*

*Fig. 10*

**Fig. 11**

**Fig. 12**

Fig. 13(a)

Fig. 13(b)

Fig. 13(c)

## Mechanical Torque vs.Current Density

**Fig. 14(a)**

**Fig. 14(b)**

Voltage Waveform with Variable Stator Length

Legend:
V @ 6A/mm²
V @ 9A/mm²
V @ 12A/mm²

Y-axis: Voltage (V)
X-axis: Time (msec)

*Fig. 14(c)*

Mechanical Torque vs. Slot Pitch

**Fig. 15(a)**

Efficiency and Loss vs. Slot Pitch

**Fig. 15(b)**

Voltage Waveform with Variable Slot Pitch

V @ 50%
V @ 70%
V @ 90%

*Fig. 15(c)*

Fig. 16A

Fig. 16B

Fig. 16C

Fig. 16D

*Fig. 16E*

*Fig. 17*

EP 3 437 177 B1

*Fig. 18*

FIG. 19

FIG. 20

*Fig. 21A*

EP 3 437 177 B1

*Fig. 21B*

EP 3 437 177 B1

*Fig. 22A*

*Fig. 22B*

*Fig. 22C*

**Fig. 23**

**Fig. 24**

Magnetic Field at the Center of Drive Magnet for Different Impeller Magnet Positions

*Fig. 25A*

Magnetic Field 0.125" from the Center of Drive Magnet for Different Impeller Magnet Positions

Fig. 25B

EP 3 437 177 B1

Fig. 25C

EP 3 437 177 B1

MIXER/PUMP/
BIOREACTOR SYSTEM

902 — CONTROLLER
(CONTROLS
MIXER/PUMP
DRIVE)

904 — DRIVE
(STATOR,MOTOR,
MAG COUPLING
,...ETC.)

906 — IMPELLER
(BLADES)

900

SENSOR(S) 907

TOURQE/SPEED
SENSOR
908

CURRENT/
VOLTAGE/
FLUX SENSOR
910

ANALYZER 912

TOURQE/SPEED
CALCULATION POWER
TO FLUID (T*n) FLUID
PROPERTIES (DENSITY,
VELOCITY) MIXING
PROPERTIES (CHANGE
IN FLUID PROPERTIES,
ABNORMALITIES IN
MIXING, BLOCKAGE,
GAS DISPERSION...ETC.)

FIG. 26

EP 3 437 177 B1

*Fig. 27*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1188474 A1
- US 2015259638 A1
- JP 2008086115 A
- CN 105327639 A
- EP 2813281 A1